# DEMANDE DE BREVET EUROPEEN

(11) **EP 2 562 154 A1**
(43) Date de publication de la demande: **27.02.2013**
(21) Numéro de dépôt: 12172410.8
(22) Date de dépôt: 27.02.2009
(51) Int. Cl.: C07C 213/10, C07C 217/84, C07C 231/16, C07C 237/08

(54) **Procédé de preparation de combretastatine**

(30) Priorité: 28.02.2008 FR 0801092
(62) Demande divisionnaire de: 09725421.3
(71) Demandeur: SANOFI, 75008 Paris (FR)
(72) Inventeur: Frederic, Marc, 75008 Paris (FR); Lutz, Sylviane, 75008 Paris (FR); Malpart, Joël, 75008 Paris (FR); Masson, Philippe, 75008 Paris (FR); Mutti, Stéphane, 75008 Paris (FR)
(74) Mandataire: Gaslonde, Aude

(57) **Abrégé**

L'invention est relative à un procédé d'enrichissement du sel du composé amino de formule : 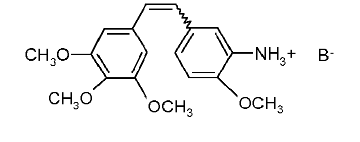 en isomère (Z).

## Description

La présente demande est relative à un procédé de préparation d'une combrétastatine **(A) :** 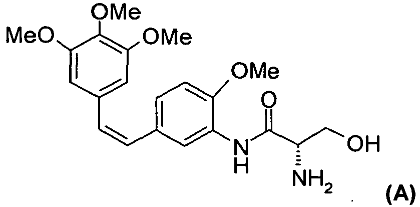 sous forme de base ou d'un sel d'addition avec un acide.

### [Art antérieur]

US 6759555 décrit un procédé de préparation d'une combrétastatine de formule : 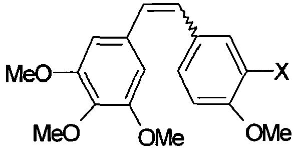 dans laquelle X représente -NH₂ ou l'un des deux groupes suivants : 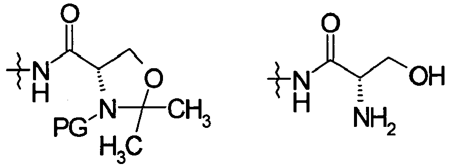 PG désignant un groupe protecteur de la fonction amine.

Bioorg. Med. Chem. 2000, 8, 2417-2425 et US 200310220404 décrivent également des procédés de préparation de combrétastatines.

J. Pept. Res. 1999, 54(1), 54-65 compare des activateurs d'acide dans la formation de liaisons peptidiques et conclut que le TDBTU est le meilleur.

Bioorg. Med. Chem. 2006, 14, 3231-3244 décrit la préparation de la combrétastatine de formule **(A)** avec un couplage utilisant comme activateur d'acide, le DCC, HOBt-H₂O (étape e, composé 10).

Chem. Commun. 1999, 1847-1848 décrit le T3P dans la préparation de liaisons amide; cependant, le T3P est décrit comme conduisant à plus d'épimérisation et à des rendements moins bons que le HAPyU (voir tableau 3).

Dans ces documents, le procédé faisant l'objet de la revendication 1 n'est pas décrit ni suggéré. De même, l'enrichissement du sel du composé amino à l'aide d'alcool benzylique et d'acétonitrile ainsi que le couplage à l'aide du T3P ne sont pas décrits.

### [Le problème technique]

Les combrétastatines ou dérivés du stilbène présentent une forte activité cytotoxique et sont de ce fait utilisables comme anticancéreux. Cependant, ce sont les isomères (Z) qui présentent la plus forte activité cytotoxique. Ces composés sont notamment décrits dans US 5731353**,** US 5561122 **ou** US 6759555**.** La Demanderesse a amélioré le procédé de préparation de la combrétastine **(A).**

### [Brève description de l'invention]

L'invention est relative à un procédé de préparation d'une combrétastatine **(A) :** 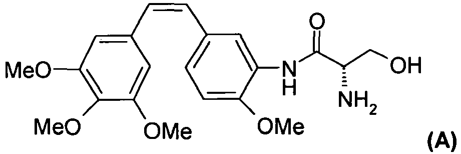 sous forme de base ou d'un sel d'addition avec un acide, consistant à coupler en présence d'une base et de T3P de formule (III) : 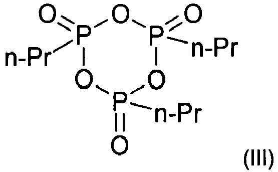 le composé (Z)-amino 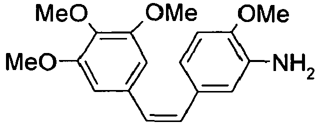 ou le sel du composé (Z)-amino 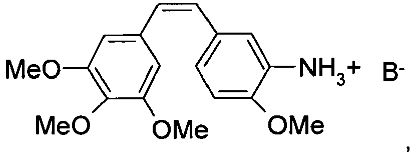 B⁻ désignant un contre-anion, avec un dérivé de la L-sérine doublement protégé de formule 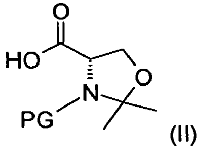 dans lequel PG désigne un groupe protecteur de la fonction amine de façon à obtenir le composé de formule (Z)-(Ib) : 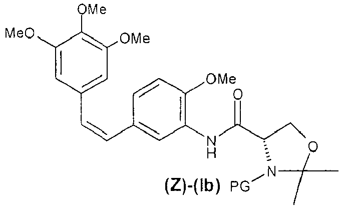 puis à déprotéger et ouvrir le cycle de (Z)-(Ib) en présence d'un acide de façon à obtenir la combrétastatine **(A)** sous forme de sel (-NH₃⁺) et éventuellement à rajouter une base de façon à obtenir la combrétastatine **(A)** sous forme de base (-NH₂),
le sel du composé (Z)-amino ayant été obtenu par un enrichissement du sel du composé amino de formule : 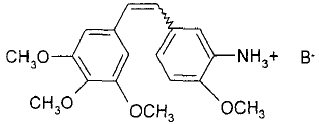 en isomère (Z), B⁻ désignant un contre-anion, consistant :
- à ajouter de d'alcool benzylique à une suspension d'un mélange des sels des composés (Z)- et (E)-amino dans l'acétonitrile puis
- à séparer mécaniquement le sel du composé amino enrichi en isomère (Z).

L'invention est aussi relative à l'enrichissement du sel du composé amino de formule 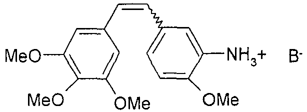 en isomère (Z), B⁻ désignant un contre-anion, consistant :
- à ajouter de d'alcool benzylique à une suspension d'un mélange des sels des composés (Z)- et (E)-amino dans l'acétonitrile et
- à séparer mécaniquement le sel du composé amino enrichi en isomère (Z).

L'invention est aussi relative à l'utilisation du T3P de formule (III) 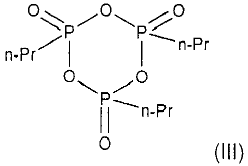 pour réaliser en présence d'une base le couplage entre le composé (Z)-amino ou le sel du composé (Z)-amino avec le dérivé de la L-sérine doublement protégé.

La proportion acétonitrile / sels des composés (Z)- et (E)-amino exprimée en poids est comprise entre 5 et 17, de préférence entre 10 et 12. La température à laquelle est conduit l'enrichissement est comprise de préférence entre 20 et 70°C. La proportion alcool benzylique /sels des composés (Z)- et (E)-amino exprimée en poids est comprise entre 1 et 4, de préférence entre 2 et 3.

### [Description détaillée de l'invention]

Le **Schéma 1** ci-dessous décrit les étapes réactionnelles (i) à (iv) d'un procédé de préparation de la combrétastatine **(A)** : 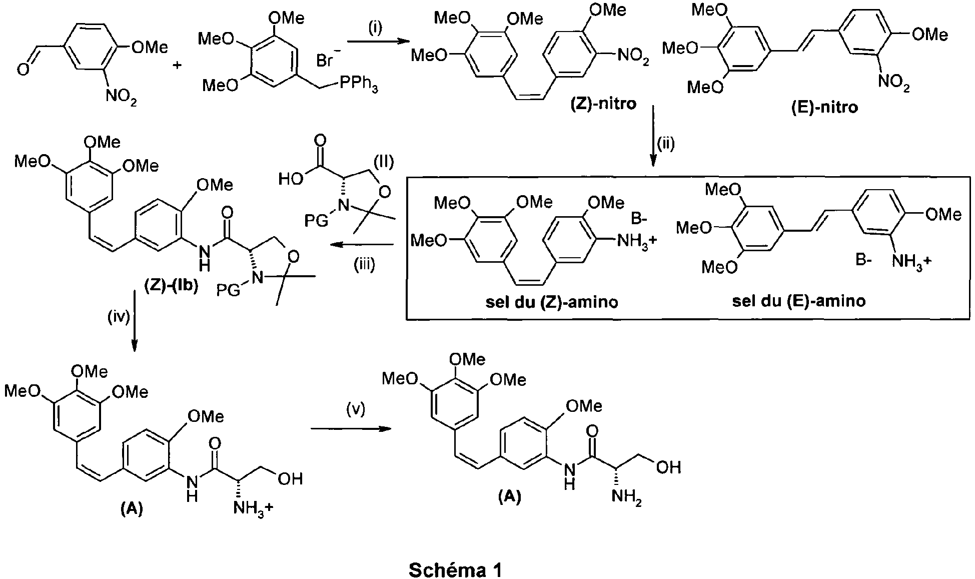
- **étape (i)** :: réaction de Wittig entre le nitrométhoxybenzaldéhyde et le bromure ou chlorure de triméthoxybenzylphosphonium en présence d'une base conduisant au sel du composé nitro, se présentant sous forme d'un mélange des deux isomères (Z) et (E) du 2-méthoxy-5-[2-(3,4,5-triméthoxyphényl)vinyl]nitrobenzène ((Z)- et (E)-nitro) ;
- **étape (ii)** :: réduction du mélange conduisant à un mélange des composés (Z)- et (E)-amino qui sont transformés en sels (B⁻ désigne un contre-anion par exemple Cl⁻ ou SO₄²⁻), suivie d'une étape de séparation du composé (Z)-amino ;
- **étape (iii)** :: couplage du sel du composé (Z)-amino avec un dérivé de la L-sérine doublement protégé sur les fonctions -OH et amino (composé de formule (II)) conduisant au composé (Z)-(Ib). 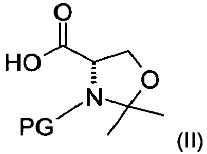
PG désigne un groupement protecteur de la fonction amine : il peut s'agir par exemple du tert-butoxycarbonyle (BOC), du benzyloxycarbonyle (CBZ) ou du 9-fluorénylméthyloxycarbonyle (FMOC) ;

### étapes (iv) et (v) : déprotection et ouverture du cycle conduisant à la combrétastatine (A) sous forme salifiée ou non.

L'étape (i) est décrite dans US 5731353 ainsi que dans *Bioorg. Med. Chem.* **2000,** *8*, 2417-2425 **(Schéma 1)**. La réaction est conduite dans un solvant organique tel que par exemple un solvant aromatique (par ex. toluène) en présence d'une base, de préférence forte telle que MeONa ou NaH. Le rapport (Z)/(E) est de l'ordre de 75/25.

L'étape (ii) de réduction décrite dans US 6759555 est conduite en présence de fer en excès (plus de 2 équivalents par rapport à (la)). La réduction en présence de zinc telle que décrite dans US 5525632 (ex.2, étape 2) est également possible mais elle n'est pas complète (rdt=49,3%) et elle conduit de plus à la formation d'une quantité importante de sous-produit « azo ». Le composé (Z)-amino d'une pureté suffisante est ensuite obtenu par une ou plusieurs séparation(s) compliquée(s). La séparation qui suit la réduction est réalisée par cristallisations successives. Une 1^{ère} cristallisation permet d'éliminer le composé (E)-amino, puis une 2^{ème} cristallisation permet d'isoler le (Z)-amino (voir US 6759555**,** ex.1).

Le couplage de l'étape (iii) est réalisé avantageusement en présence d'un activateur d'acide tel que par exemple l'EDCI (chlorure de 1-(3-diméthylaminopropyl)-3-éthylcarbodiimide), le DCC (dicyclohexylcarbodiimide), le TOTU (o-[éthoxycarbonyl]cyanométhylèneamino)-N,N,N',N'-tétraméthyluronium tétrafluoroborate), le HBTU (o-benzotriazol-1-yl-N,N,N',N'-tétraméthyluronium hexafluorophosphate), le PivCl (chlorure de pivaloyle) ou le N,N-carbonyldiimidazole. On désigne par activateur d'acide (« coupling agent ») un composé dont la fonction est d'activer la fonction acide -COOH afin de favoriser la formation d'une liaison peptidique. Pour plus de détails sur les activateurs d'acide, on pourra se référer à la revue ChemFiles Vol.7, N°2, page 3 éditée par la société Aldrich Chemical ou bien à Tetrahedron report N°672, 2004, 60, 2447-2467, « Recent development of peptide coupling reagents in organic synthesis ». La revue Tetrahedron 2005, 61, 10827-10852 révèle qu'il existe beaucoup d'activateurs d'acide disponibles.

L'étape (iv) est conduite en présence d'un acide afin de favoriser l'ouverture de cycle et d'obtenir la combrétastatine **(A)** sous forme d'un sel (-NH₃⁺). Il s'agit d'une déprotection/ouverture de cycle.

En présence de BOC, on utilise avantageusement l'acide chlorhydrique, par exemple sosu forme de solution méthanolique, et on obtient le chlorhydrate. La combrétastatine **(A)** sous forme de base est obtenue en neutralisant le sel à l'aide d'une base, par exemple la soude (cf.ex.1-étape (iii)).

### Procédé selon l'invention

Le procédé de la présente invention reprend les mêmes étapes du **Schéma 1** mais les étapes (ii) et (iii) ont été améliorées. En effet, dans l'invention, au cours de l'étape (ii), l'isomère (Z) du sel du composé amino est obtenu par le procédé consistant :
- à ajouter de d'alcool benzylique à une suspension d'un mélange des sels des composés (Z)- et (E)-amino dans l'acétonitrile et
- à séparer mécaniquement le sel du composé amino enrichi en isomère (Z).

L'isomère (Z) est donc obtenu par « enrichissement », ce terme signifiant qu'à l'issue des deux étapes du procédé ci-dessus décrites, on augmente la proportion de l'isomère (Z) par rapport à l'isomère (E) ; on peut également parler de procédé de séparation de l'isomère (Z) à partir d'un mélange des isomères (Z) et (E)). Par rapport à une (re)cristallisation, l'enrichissement présente l'avantage d'être à la fois direct et simple à mettre en oeuvre. Il permet d'obtenir le composé amino enrichi en isomère (Z) avec une faible teneur en isomère (E) résiduel (pouvant aller jusqu'à <1 % mol ; cf. ex.1 où la pureté du produit est de 99,93% en (Z) et 0,07% en (E)). La séparation mécanique peut être par exemple une filtration ou une centrifugation. A l'issue de la séparation mécanique, le sel de l'isomère (Z) peut être éventuellement lavé et séché.

La suspension présente de préférence une proportion acétonitrile / sels des composés (Z)- et (E)-amino exprimée en poids comprise entre 5 et 17, de préférence entre 10 et 12 (c'est-à-dire que le poids d'acétonitrile dans la suspension est compris entre 5 à 17 x poids des composés (Z)- et (E)-amino).

La quantité d'alcool benzylique qui est ajoutée est de préférence telle que la proportion alcool benzylique / sels des composés (Z)- et (E)-amino exprimée en poids est comprise entre 1 et 4, de préférence entre 2 et 3 (c'est-à-dire que le poids d'alcool benzylique ajouté est compris entre 1 à 4 x poids des composés (Z)- et (E)-amino). Cette proportion permet de maintenir un rapport (Z)/(E) élevé pour le produit final. L'alcool benzylique a pour fonction de dissoudre préférentiellement le sel du composé (E)-amino.

L'enrichissement est mis en oeuvre de préférence à une température comprise entre 20 et 70°C, avantageusement entre 30 et 70°C, de préférence entre 35 et 65°C. Au-delà de 70°C, le composé amino commence à se dégrader lentement.

Une méthode préférée de préparation de la suspension est décrite ci-après. On réduit les composés (Z)- et (E)-nitro à l'aide de dithionite de sodium (Na₂S₂O₄) dans un solvant qui peut être un mélange d'eau et d'alcool, par exemple un mélange eau/méthanol. Après la réduction, on introduit dans le milieu réactionnel un acide fort (par ex. HCl ou H₂SO₄) qui réagit avec les intermédiaires de la réaction ainsi qu'avec les restes de dithionite et de disulfite. On obtient alors un mélange des sels des composés (Z)- et (E)-amino (par ex. chlorhydrate ou sulfate). On ajoute alors une base forte pour obtenir les bases libres (-NH₃⁺ ⇒ -NH₂) qui sont extraites par un solvant organique, par exemple un solvant chloré tel que le dichlorométhane (DCM). On ajoute à la phase organique un acide fort dans un alcool, puis on déplace l'alcool par de l'acétonitrile de façon à obtenir une suspension des sels des composés (Z)- et (E)-amino dans l'acétonitrile. L'acide fort peut être HCl ou H₂SO₄ (B⁻ = Cl ou SO₄⁻). Le déplacement de solvant peut se faire en ajoutant l'acétonitrile après avoir éliminé sous vide plus ou moins complètement l'alcool. L'ajout d'acétonitrile peut se faire également concomitamment avec l'élimination de l'alcool sous vide. L'alcool est de préférence un alcool léger tel que le méthanol ou l'éthanol.

Pour l'étape (iii), le couplage entre le sel du composé (Z)-amino et le dérivé de la L-sérine doublement protégé de formule (II), est réalisé dans un solvant organique à l'aide de l'anhydride de l'acide propane phosphonique (T3P) comme activateur d'acide en présence d'une base. La fonction de la base est de piéger les espèces acides et de déplacer le sel vers la base libre. La quantité de base ajoutée est comprise entre 2 et 3 éq. par rapport au sel du composé (Z)-amino (voir ex.1 où l'on a utilisé 2,7 éq.). Il est possible également de coupler directement le composé (Z)-amino avec le composé de formule (II) en présence de T3P et d'une base ; dans ce cas, la proportion de base ajoutée est moins importante, comprise entre 1 et 2 éq. (environ 1 éq. de moins que si on part du sel).

La base peut être une amine tertiaire comme par exemple la triéthylamine (TEA), la diisopropyléthylamine (DIEA), la N-méthyl morpholine (NMM) ou la méthyl-pipéridine. Le solvant organique peut être le dichlorométhane (DCM), le toluène, la méthylisobutyl cétone (MIBK), l'acétate d'éthyle, l'acétonitrile, le tétrahydrofurane (THF), le Me-tétrahydrofurane (Me-THF) ou le cyclopentylméthyl éther.

Le T3P a pour formule :

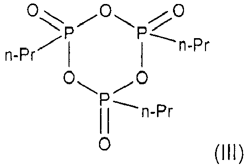

L'avantage à utiliser le T3P plutôt qu'un autre activateur d'acide est que les sous-produits du T3P sont facilement éliminables (sous-produits tous solubles dans l'eau) et qu'il s'agit d'un activateur bon marché. De plus, la réaction peut être conduite en présence du composé (Z)-amino (sous forme de sel ou de base) et du composé (II) mis ensemble selon un procédé « one pot » : on fait donc réagir ensemble dans le même récipient le composé (Z)-amino (sous forme de sel ou de base), le composé (II) en présence du T3P et d'une base. Cela n'est pas le cas de tous les agents de couplage car certains, tel que le PivCl (chlorure de pivaloyle), nécessitent pour la même réaction une activation préalable de la fonction acide du composé (II) avant de pouvoir ajouter le sel du composé (Z)-amino.

Enfin, nous avons constaté que le T3P ne conduit pas à une épimérisation du centre asymétrique, ce qui permet d'obtenir la combrétastatine **(A)** avec une bonne pureté et un bon rendement. On a constaté aussi que le T3P permet d'obtenir un bon rendement en produit de couplage (voir tableau III).

La réaction de couplage est conduite généralement à une température comprise entre 5°C et 70°C, par exemple au reflux du DCM. La proportion de T3P par rapport au composé (Z)-amino est comprise entre 1 et 2 éq, de préférence entre 1,5 et 1,8 éq.

### [Exemples]

### Exemple 1 (selon l'invention)

### Etape (i) :

Sur un mélange à une température de 5-10°C, constitué de toluène (91,1 litres) de bromure de triméthoxybenzylphosphonium (15,35 kg, 29,33 moles) et de 4-méthoxy-3-nitrobenzaldéhyde (5,06 kg, 27,93 moles), est coulée une solution de méthylate de sodium (5,66 kg, 29,34 moles). En fin de réaction, 0,32 litre (5,59 moles) d'acide acétique est coulé. Après un maintien à 20°C, le milieu est filtré. Le gâteau est lavé avec du toluène (11,1 litres). Les filtrats sont lavés plusieurs fois avec de l'eau (20,2 litres) puis concentrés sous vide. L'alcool isopropylique (87,6 litres) est alors chargé et le milieu est concentré puis refroidi. La suspension est alors filtrée à 10°C. Le produit isolé est séché sous vide (6,46 kg, 52,2%). La pureté du produit isolé est de l'ordre de 78% en (Z) et de 22% en (E).

### Etape (ii) :

Sur un milieu constitué de méthanol (100 ml), les composés (Z) et (E)-nitro à réduire (20 g, 0,058 mole) de dithionite de sodium (36,8 g, 0,179 mole, 3,1 éq), est coulé de l'eau (80 ml) à 50°C. Une fois la réaction terminée (on a laissé 1 h à 50°C), l'acide chlorhydrique (36,3 ml, 0,406 mole) est additionné. Le traitement est réalisé par coulée d'eau (70 ml) et de DCM (80 ml), puis de soude (lessive de soude 30,5%, 10N) jusqu'à pH=7. On élimine la phase aqueuse après réextraction avec du DCM (20 ml) puis la phase DCM est concentrée sous vide (environ 200 mbar à 35°C) et remplacée par l'acétonitrile (160 ml) ; on effectue le changement de solvant du DCM vers l'acétonitrile sous pression réduite de façon à garder un volume réactionnel de 200 ml environ. L'acide chlorhydrique méthanolique (27 ml, 0,081 mole) est coulé puis le solvant est évaporé sous pression réduite (90 mbar) de façon à effectuer le changement de solvant du méthanol-acétonitrile vers l'acétonitrile à volume constant d'environ 233 ml. En fin de changement de solvant, on ajuste le volume total (solvant + composés organiques) à 280 ml.

On obtient alors une suspension sous forme d'une bouillie blanche. L'alcool benzylique (46 ml) est alors ajouté à la suspension à 45±3°C. Après refroidissement vers 25°C, le milieu est alors filtré et lavé avec de l'acétonitrile (30 ml) et de l'alcool benzylique (3,3 ml). Le produit isolé est alors séché sous vide (11,7 g, 74,4%). Ce produit présente une pureté déterminée par HPLC de 99,93 % en (Z) et 0,07% en (E). A l'issue de l'étape (ii), on a donc obtenu simplement et directement le sel du composé (Z)-amino avec une bonne pureté.

### Etape (iii) :

Sur un milieu constitué de DCM (500 ml), du composé (Z)-amino sous forme de chlorhydrate (50 g, 0,142 mole) et de la L-sérine doublement protégé de formule (II) avec PG=BOC (L-sérine-N-BOC-acétonide ; 41,8 g, 0,170 mole), est coulée de la TEA (53,4 ml, 0,383 mole) à 20°C puis une solution de T3P dans le DCM (154 g, 0,242 mole). Le milieu est porté au reflux puis de l'eau (500 ml) est additionnée. Après décantation et concentration, l'acide chlorhydrique méthanolique (189,5 ml, 1,136 moles) et du méthanol (189,5 ml) sont additionnés. De l'eau (300 ml) est ajoutée au milieu puis les phases sont décantées. L'acétate d'isopropyle (650 ml) est ajouté à la phase aqueuse puis la soude (115 ml, 1,150 moles) est coulée à 20°C. La phase organique décantée et lavée est concentrée sous vide puis chauffée à 65°C. Le méthanol (35 ml) puis l'acide chlorhydrique méthanolique (47,4 ml, 0,142 moles) sont additionnés à cette température. Après refroidissement, et filtration, le produit est isolé (49,6 g, 79,5%). Le produit final présente une pureté de 99,2%.

### Exemple 2 (selon l'invention)

On charge dans un réacteur de 1,6 litres et équipé d'une double-enveloppe, le composé (Z)-amino sous forme de chlorhydrate Z-aminostil, HCl (50,0 g), le N-BOC-acétonide (41,8 g) et 500 ml de DCM. On coule ensuite à 22±3°C 53,4 ml de triéthylamine (2,7 éq.), puis une solution de T3P dans le DCM à 50% (1,7 éq.). On agite au reflux du DCM pendant 1 heure. On refroidit ensuite le mélange à 22±3°C et on ajoute 500 ml d'eau déminéralisée. On laisse décanter et on sépare les phases. On lave la phase DCM avec 500 ml d'une solution aqueuse d'hydrogénophosphate de sodium à 6% poids (30 g), puis avec 500 ml d'eau déminéralisée. On concentre la phase DCM sous pression réduite de 360 à 150 mbar vers 40-50°C. On coule ensuite une solution HCl dans le méthanol à 3 mol/l (379 ml, 8 éq.) puis 300 ml d'eau déminéralisée. On laisse décanter et on sépare les phases. On reextrait la phase DCM/méthanol avec de l'eau déminéralisée (200 ml) et on sépare les phases. On ajoute 650 ml d'acétate d'isopropyle, puis 115 ml d'une solution d'hydroxyde de sodium à 30% (8,1 éq.). On laisse décanter et on lave la phase organique avec 400 ml d'eau déminéralisée. On laisse décanter et on sépare les phases. On concentre ensuite la phase organique sous pression réduite de 160 à 60 mbar jusqu'à 300-350 ml et on effectue un changement de solvant DCM⇒acétate d'isopropyle. On chauffe ensuite le mélange à 62°C, on ajoute 35 ml de méthanol et on coule une solution d'acide chlorhydrique dans le méthanol à 3 mol/l (47,4 ml, 1 éq.). On laisse refroidir ensuite jusqu'à l'ambiante et on filtre la bouille blanche. On lave le solide final

Dans les exemples 3-6, on reprend l'étape (ii) avec des quantités différentes de composés (Z) et (E)-nitro au départ. La proportion acétonitrile/sels des composés (Z)- et (E)-amino est fixée à 10,8 et la proportion alcool benzylique/ sels des composés (Z)- et (E)-amino est quant à elle variable.

### Exemple 3 (selon l'invention)

Sur un milieu constitué de méthanol (50 ml), les composés (Z) et (E)-nitro à réduire (15 g, 0,043 mole) de dithionite de sodium (27,2 g, 0,133 mole), est coulé de l'eau (60 ml) à 50°C. Une fois la réaction terminée, l'acide chlorhydrique (26,2 ml, 0,314 mole) est additionné. Le traitement est réalisé par coulée d'eau et du DCM, puis de soude jusqu'à pH=7. Sur la phase DCM, est additionné l'acide chlorhydrique méthanolique (18,9 ml, 0,0586 mole) puis le solvant est remplacée par l'acétonitrile. On obtient alors une bouillie. L'alcool benzylique (31 ml) est alors ajouté à la suspension à 50°C et maintenu à 65°C pendant 2 heures. Après refroidissement, le milieu est alors filtré et lavé. Le produit isolé est alors séché sous vide.

### Exemples 4-6 : identiques à l'exemple 3 mais avec une quantité différente d'alcool benzylique

**Tableau I**

| | **proportion en poids alcool benzylique / sels Z+E amino** | **pureté HPLC du Z-amino** | **rendement brut en Z-amino** |
|---|---|---|---|
| **ex.4** | 1,96 | 99,5% | 82,0% |
| **ex.3** | 2,09 | 99,6% | 81,0% |
| **ex.5** | 2,25 | 99,7% | 77,0% |
| **ex.6** | 2,38 | 99,9% | 75,6% |

| | | | |
|---|---|---|---|
| proportion acétonitrile / sels Z+E amino = 10,8 | | | |

Dans les exemples 5 et 7-9, la proportion alcool benzylique/sels des composés (Z)- et (E)-amino est fixée à 2,25 et la proportion acétonitrile/ sels des composés (Z)- et (E)-amino est variable.

**Tableau II**

| | **proportion en poids acétonitrile / sels Z+E amino** | **pureté HPLC du Z-amino** |
|---|---|---|
| **ex.7** | 9,10 | 99,7 |
| **ex.5** | 10,80 | 99,9 |
| **ex.8** | 11,88 | 98,9 |
| **ex.9** | 12,65 | 94,5 |

| | | |
|---|---|---|
| proportion alcool benzylique / sels Z+E amino = 2,25 | | |

Les exemples 10-14 décrivent des résultats de couplage (étape (iii)) utilisant d'autres agents de couplage que le T3P.

### Exemple 10 (comparatif) : utilisation du TOTU

On reprend les conditions de l'étape (iii) mais en présence de TOTU comme activateur d'acide (1 éq. de TOTU + 5 éq. de TEA). Le rendement final n'est alors que de 71 %.

### Exemple 11 (comparatif) : utilisation du TOTU

Sur un milieu constitué de DCM (10 ml), du composé (Z)-amino sous forme de chlorhydrate (0,50 g, 1,4 mmole) et de la L-sérine doublement protégé de formule (II) avec PG=BOC (L-sérine-NBOC-acétonide ; 0,35 g, 1,4 mmole), est coulé de la TEA (0,71 g, 7,0 mmole) à 5°C puis le TOTU (0,46 g, 1,4 mmole). Le milieu est maintenu à 5°C pendant 24 heures puis de l'eau (5 ml) est additionnée. Après décantation la phase organique est analysée par HPLC. Le rendement dosé du produit de couplage est de 50,1% et sa pureté est de 69,3%.

### Exemple 12 (comparatif) : utilisation du BOP-CI (Bis(2-oxo-3-oxazolidinyl)phosphinic chloride)

Sur un milieu constitué de DCM (5 ml), de composé (Z)-amino (0,50 g, 1,4 mmole) et de la L-sérine doublement protégé de formule (II) avec PG=BOC (L-sérine-NBOC-acétonide ; 0,35 g, 1,4 mmole), est coulé de la NMM (0,42 g, 4,2 mmole) à 5°C puis le BOP-CI (0,72 g, 2,8 mmole). Le milieu est maintenu à 5°C pendant 24 heures puis de l'eau (5 ml) est additionnée. Après décantation la phase organique est analysée par HPLC. Le rendement dosé du produit de couplage est de 29,1 % et sa pureté est de 91,6%.

### Exemple 13 (comparatif) : utilisation du PyCIOP (chlorotripyrrolidinophosphonium hexafluorophosphate)

Sur un milieu constitué d'acétate d'éthyle (10 ml), du-composé (Z)-amino (0,50 g, 1,4 mmole) et de la L-sérine doublement protégé de formule (II) avec PG=BOC (L-sérine-NBOC-acétonide ; 0,70 g, 2,8 mmole), est coulé de la NMN (0,42 g, 4,2 mmole) à 5°C puis le PyCIOP (1,2 g, 2,8 mmole). Le milieu est maintenu à 5°C pendant 24 heures puis de l'eau (5 ml) est additionnée. Après décantation la phase organique est analysée par HPLC. Le rendement dosé du produit de couplage est de 53,3% et sa pureté est de 83,9%.

### Exemple 14 (comparatif) : utilisation du PyBROP (bromo-tris-pyrrolidino phosphoniumhexafluorophosphate)

Sur un milieu constitué de Me-CN (5 ml), du-composé (Z)-amino (0,50 g, 1,4 mmole) et de la L-sérine doublement protégé de formule (II) avec PG=BOC (L-sérine-NBOC-acétonide ; 0,35 g, 1,4 mmole), est coulé de la NMN (0,42 g, 4,2 mmole) à 25°C puis le PyBROP (0,65 g, 1,4 mmole). Le milieu est maintenu à 25°C pendant 24 heures puis de l'eau (5 ml) est additionnée. Après décantation la phase organique est analysée par HPLC. Le rendement dosé du produit de couplage est de 24,9% et sa pureté est de 75,4%.

**Tableau III**

| | **agent de couplage** | **base** | **rendement dosé du produit de couplage (Z)-(Ib)** | **pureté par HPLC** |
|---|---|---|---|---|
| **ex.2** | T3P | TEA - 2,7 éq. | 99,7% | - |
| **ex.11** | TOTU | TEA - 5 éq. | 50,1% | 69,3% |
| **ex.12** | BOP-CI | NMN - 3 éq. | 29,1% | 91,6% |
| **ex.13** | PyCIOP | NMN - 3 éq. | 53,3% | 83,9% |
| **ex.14** | PyBROP | NMN - 1 éq. | 24,9% | 75,4% |

On constate que le T3P permet d'obtenir un meilleur rendement en produit de couplage que le TOTU, BOP-CI, PyCIOP ou le PyBROP.

## Revendications

1. Procédé d'enrichissement du sel du composé amino de formule 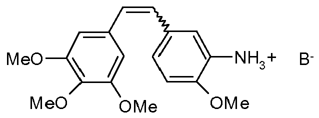 en isomère (Z), B⁻ désignant un contre-anion, consistant :
• à ajouter de d'alcool benzylique à une suspension d'un mélange des sels des composés (Z)- et (E)-amino dans l'acétonitrile et
• à séparer mécaniquement le sel du composé amino enrichi en isomère (Z).

2. Procédé selon la revendication 1 dans lequel la séparation mécanique est une filtration.

3. Procédé selon l'une des revendications 1 ou 2 dans lequel B⁻ désigne Cl⁻ ou SO₄²⁻.

4. Procédé selon l'une des revendications 1 à 3 dans lequel la proportion acétonitrile / sels des composés (Z)- et (E)-amino exprimée en poids est comprise entre 5 et 17.

5. Procédé selon l'une des revendications 1 à 3 dans lequel la proportion acétonitrile / sels des composés (Z)- et (E)-amino exprimée en poids est comprise entre 10 et 12.

6. Procédé selon l'une des revendications 1 à 5 dans lequel la température à laquelle est conduit l'enrichissement est comprise entre 20 et 70°C.

7. Procédé selon l'une des revendications 1 à 6 dans lequel la proportion alcool benzylique /sels des composés (Z)- et (E)-amino exprimée en poids est comprise entre 1 et 4.

8. Procédé selon l'une des revendications 1 à 6 dans lequel la proportion alcool benzylique /sels des composés (Z)- et (E)-amino exprimée en poids est comprise entre 2 et 3.

9. Procédé selon l'une des revendications 1 à 8, dans lequel la suspension du mélange des sels des composés (Z)- et (E)-amino est obtenue par réduction des composés (Z)- et (E)-nitro de formule suivante : 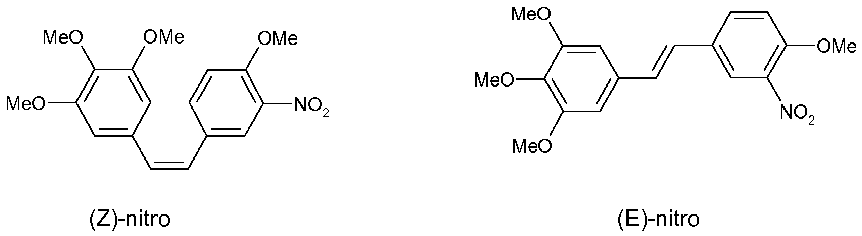 avec le dithionite de sodium (Na₂S₂O₄) dans un solvant.

10. Procédé selon la revendication 9, dans lequel le solvant est un mélange eau/méthanol.
